# EUROPEAN PATENT APPLICATION

(11) **EP 4 745 243 A1**
(43) Date of publication of application: **20.05.2026**
(21) Application number: 24212682.9
(22) Date of filing: 13.11.2024
(51) Int. Cl.: C12P 7/625, C12N 1/20, C12N 1/36, C12P 7/6409, C12R 1/05

(54) **FERMENTATION PROCESS FOR PRODUCING POLYHYDROXYALKANOATE**

(71) Applicant: NG Nordic Finland Oy, 11120 Riihimäki (FI)
(72) Inventor: HUUSELA, Martina, 11120 Riihimäki (FI); PAWAR, Sudhanshu, 69285 Kumla (SE)
(74) Representative: Laine IP Oy

(57) **Abstract**

According to an example aspect of the present invention, there is provided a continuous fermentation process for producing at least one polyhydroxyalkanoate. The process comprises the steps of: producing a fermentation broth comprising a biomass in at least one primary bioreactor, and passing the fermentation broth through secondary bioreactors for producing at least one polyhydroxyalkanoate. In the process the biomass comprises at least one micro-organism capable of producing the at least one polyhydroxyalkanoate, and pH within the bioreactors is maintained in the range of 6 to 8 by addition of a carbon source and a nitrogen source. The carbon source is a volatile fatty acid or a mixture of volatile fatty acids, and the nitrogen source is ammonia, ammonium hydroxide, or an ammonium salt or a combination thereof, and the carbon source and the nitrogen source are added to the at least one primary bioreactor in a molar ratio ranging from 5:1 to 30:1, and to the secondary bioreactors in a molar ratio ranging from 70:1 to 150:1.

## Description

### FIELD

The invention belongs to the field of polymer production. More specifically the invention relates to production of biopolymers, specifically to production of polyhydroxyalkanoates by microbial fermentation.

### BACKGROUND

Need for plastics is expected to increase to 1100 Mt by 2050 and is expected to contribute to 19% of the world's greenhouse gas emission. Even when 63% of this plastic is expected to be recycled, there are limitations in current recycling methods resulting in residual plastics waste that is either too complex or too hazardous to be recycled with current methodology. Today 90% of the plastics is produced from fossil sources. Only 9% is recycled due to the challenges with chemical (pyrolysis) and mechanical recycling. Thus, plastics are mostly landfilled while part is incinerated for energy recovery.

Biodegradable polymers are an alternative to the regular fossil fuel -based plastics. However, production of durable bioplastics is still challenging and expensive and thus implementation of bioplastics is still limited. New, improved production methods are thus needed.

### SUMMARY OF THE INVENTION

The invention is defined by the features of the independent claims. Some specific embodiments are defined in the dependent claims.

According to a first aspect, there is provided a continuous fermentation process for producing at least one polyhydroxyalkanoate. The process comprises the steps of:
- producing a fermentation broth comprising a biomass, in at least one primary bioreactor;
- passing the fermentation broth through secondary bioreactors for producing at least one polyhydroxyalkanoate;
wherein
- the biomass comprises at least one micro-organism capable of producing the at least one polyhydroxyalkanoate;
- pH within the bioreactors is maintained in the range of 6 to 8 by addition of a carbon source and a nitrogen source, wherein
   - the carbon source is a volatile fatty acid or a mixture of volatile fatty acids, and the nitrogen source is ammonia, ammonium hydroxide, an ammonium salt or a combination thereof;
   - the carbon source and the nitrogen source are added to the at least one primary bioreactor in a molar ratio ranging from 5:1 to 30:1, and to the secondary bioreactors in a molar ratio ranging from 70:1 to 150:1.

Various embodiments of the first aspect may comprise at least one feature from the following bulleted list:
- the volatile fatty acid is selected from acetic acid, propionic acid, butyric acid, valeric acid, isovaleric acid, hexanoic acid, or combinations thereof.
- the volatile fatty acid and the ammonia, ammonium hydroxide, ammonium salt or combination thereof are the sole carbon source and the sole nitrogen source, respectively, for the at least one micro-organism.
- a fermentation medium comprising metal ions and salts and depleted of the carbon source and the nitrogen source is introduced as a feed separate from the carbon source and the nitrogen source.
- the total amount of volatile fatty acids is less than 10 g/L in the fermentation broth at any point during the fermentation process.
- the carbon source and the nitrogen source are added as a solution comprising at least 30 %, such as at least 50 % of at least one volatile fatty acid of the weight of the solution.
- the fermentation broth is passed through two to five secondary bioreactors.
- the process is configured to maintain a continuous flow from a preceding bioreactor to a subsequent bioreactor.
- the fermentation broth is continuously extracted from at least one of the secondary bioreactors during or after the formation of the at least one polyhydroxyalkanoate at a rate allowing the volume of the fermentation broth in the at least one primary bioreactor and in the secondary bioreactors to change less than 5 % of the initial volume in the bioreactors.
- the biomass is separated from the fermentation broth after the extraction from the at least one secondary bioreactor.
- the at least one polyhydroxyalkanoate is formed by the polymerization of 3-hydroxyvalerate, 3-hydroxybutyrate, 3- hydroxyhexanoate, 3-hydroxypropionate, or combinations thereof.
- the mixture of volatile fatty acids comprises at least 1 %, such as at least 5 %, of valeric acid, isovaleric acid, and/or hexanoic acid of the weight of the mixture of volatile fatty acids.
- the mixture of volatile fatty acids is produced from carbon dioxide and hydrogen gas by using at least one microorganism expressing the enzymes of the Wood-Ljungdahl pathway.
- the carbon source and the nitrogen source are added to the at least one primary bioreactor in a molar ratio ranging from 10:1 to 25:1 and to the secondary bioreactors in a molar ratio ranging from 70:1 to 90:1.

According to a second aspect, there is provided a product obtainable with the process according to the first aspect of the invention.

### Some advantages

An advantage of the present disclosure is that it allows a continuous process for producing bioplastics. With the continuous process a stable production of bioplastics may be obtained and the process can be highly automized making it predictable and cost-efficient.

Another advantage of at least some embodiments of the present disclosure is that it allows simultaneous control of the pH of the fermentation broth and control over production of biomass and polyhydroxyalkanoates.

Another advantage of the present disclosure is that it allows minimising the use of chloride, nitrogen and sulphates in the fermentation process. These compounds and ions are known to be difficult in industrial processes and thus minimizing their use for example in pH control is very appealing. For example, sulphates used in fermentation processes lead to formation of H₂S which is corrosive and nitrogen ions form an environmental burden.

Another advantage of at least some embodiments of the present disclosure is that it provides a process in which contamination can be minimized. This may be achieved by using volatile fatty acids as the carbon source and thus microbial growth in the feed can be inhibited by increasing the acid content of the feed to such level that it inhibits microbial growth.

Another advantage of at least some embodiments of the present invention is that it allows reducing and even excluding the typical pH control agents, such as HCl, NaOH, KOH, HNO₃ and/or H₂SO₄, from a microbial fermentation process.

### BRIEF DESCRIPTION OF THE DRAWINGS

FIGURE 1 illustrates pH control in a primary reactor. The variables are depicted as follows: solid line at bottom= feeding event of CN solution, solid circle at top= measured pH, solid square in the middle = pH set-point.
FIGURE 2 illustrates pH control in a secondary bioreactor. The variables are depicted as follows: solid line at the bottom= feeding event of CN solution, solid circle at the top= measured pH, solid square in the middle = pH set-point.
FIGURE 3 illustrates FTIR absorbance profile of samples from the primary (R1) and secondary bioreactors (R2 and R3) during process time 60 hours. Circle has been placed around the peak at 1744 cm⁻¹ showing accumulation of polyhydroxyalkanoates.

### EMBODIMENTS

### DEFINITIONS

In the present context, the terms "polyhydroxyalkanoates" or "PHA" refer to a group of polyesters produced in the nature by numerous microbes. Typically, they are fermented from sugars or lipids. More than 150 different monomers can be combined within this family to give materials with different properties. The formed polyesters are biodegradable and can be used in the production of bioplastics. Examples of polyhydroxyalkanoates include poly-3-hydroxyvalerate, poly-4-hydroxybutyrate, (poly(3-hydroxybutyrate-co-3-hydroxyvalerate), poly(3-hydroxyoctanoate) and poly(3-hydroxynonanoate).

In the present context the term "fermentation" refers to a process of applying microbes for the production of compounds. In a typical process, a liquid medium is inoculated with a microbial culture and the microbes are grown under specific conditions. At a suitable timepoint, the production of the desired chemical may be induced. Such induction is typically done by addition of a chemical, and/or by changing conditions, such as temperature, feed strategy and/or pressure. The liquid medium comprises nutrients that allow the microbial culture to grow and may be optimized for the used microbial culture. The microbial culture typically comprises bacterial and/or yeast cells in a monoculture or in a polyculture. A monoculture consists of a single microbial species whereas a polyculture may comprise multiple different microbial species. A fermentation process may be aerobic or anaerobic.

In the present context, the term "culture" refers to a population of unicellular or multicellular microorganisms in a medium, such as a growth or fermentation medium. The term "biomass" refers to a population of unicellular or multicellular microorganisms.

In the present context, the term "fermentation broth" refers to a composition produced during microbial fermentation. Fermentation broth comprises liquid medium (also known as fermentation medium), which is provided to a bioreactor and comprises for example nutrients; and biomass that is produced during fermentation from a microbial culture provided to the bioreactor at the beginning of the microbial fermentation. The fermentation broth further comprises products produced during the fermentation by chemical reactions and/or by the micro-organisms.

In the present context, the terms "volatile fatty acids" or "VFAs" refer to short chain fatty acids that are water soluble and may be separated by steam distillation at atmospheric pressure. Volatile fatty acids typically comprise carbon backbones of 1 to 6 carbon atoms. Examples of volatile fatty acids include but are not limited to acetic acid, propionic acid, butyric acid, valeric acid, isovaleric acid, hexanoic acid and succinic acid.

In the present context, the term "continuous fermentation process" refers to an open fermentation system in which a continuous influx of fresh nutrients and continuous efflux of the spent media occurs. Biomass and fermentation products are continuously produced in the system. A continuous fermentation process differs from a fed-batch fermentation process in which one or more nutrients are supplied to a bioreactor during cultivation of micro-organisms but the product(s) remain in the bioreactor until the end of the process. Batch fermentation process is performed in a closed system where as continuous fermentation process is performed in an open system. This difference affects for example contamination risks, nutrient use, and waste management and thus such processes, especially their control mechanisms, are not directly comparable.

The present disclosure provides a continuous fermentation process for the production of polyhydroxyalkanoates. Carbon source and nitrogen source are provided to a fermentation medium or fermentation broth in a controlled manner so that the pH of the system is simultaneously controlled with controlling the growth of a biomass and production of polyhydroxyalkanoates. The disclosed process allows reducing and even excluding the typical pH control agents, such as HCl, NaOH, KOH, HNO₃ and/or H₂SO₄, which are problematic in industrial processes. In addition, the process disclosed herein allows reducing the use of sulphates in the fermentation processes and thus reduce the amount of corrosive H₂S production.

The herein disclosed process is a continuous fermentation process for producing at least one polyhydroxyalkanoate. The process comprises the steps of producing a fermentation broth comprising a biomass in at least one primary bioreactor, and passing the fermentation broth through secondary bioreactors for producing at least one polyhydroxyalkanoate. In the process, the biomass comprises at least one micro-organism capable of producing the at least one polyhydroxyalkanoate; and the pH within the bioreactors is maintained in the range of 6 to 8 by addition of a carbon source and a nitrogen source. The carbon source is a volatile fatty acid or a mixture of volatile fatty acids, and the nitrogen source is ammonia, ammonium hydroxide, an ammonium salt or a combination thereof; and the carbon source and the nitrogen source are introduced to the at least one primary bioreactor in a molar ratio ranging from 5:1 to 30:1, and to the secondary bioreactors in a molar ratio ranging from 70:1 to 150:1.

Typically, the at least one primary bioreactor is operated at conditions to primarily promote microbial growth and the secondary bioreactors are operated at conditions to primarily produce at least one polyhydroxyalkanoate. However, biomass production is not limited only to the at least one primary bioreactor but some biomass production may also take place in the secondary bioreactors. Similarly, although the at least one polyhydroxyalkanoate is primarily produced in secondary bioreactors, some polyhydroxyalkanoate production may take place already in the at least one primary bioreactor.

The at least one micro-organism comprised in the biomass is typically a bacterium. The micro-organism must be able to produce the at least one polyhydroxyalkanoate. Non-limiting examples of suitable micro-organisms include *Cupriavidus necator, Methylorubrum extorquens, Paracoccus denitrificans, Methylobacterium organophilum, Pseudomonas aeruginosa, Bacillus megaterium, Halomonas bluephagenensis and other Halomonas species, Haloferax species,* and *Escherichia coli.* In some embodiments, the micro-organism may be a genetically modified micro-organism so that it has become capable of producing the at least one polyhydroxyalkanoate. In some examples, the biomass may comprise a single micro-organism, i.e. the biomass is a monoculture. In other examples the biomass may comprise a co-culture comprising 2, 3 or 4 microbial species. A monoculture refers to a microbial culture comprising only a single microbial species.

pH of a fermentation medium or a fermentation broth is controlled during a fermentation process because pH inevitably changes when microbes grow and at some point, the pH is no longer optimal for production of biomass and/or for the production of polyhydroxyalkanoates. It has been discovered that when volatile fatty acids are used as a carbon source, the carbon source can be simultaneously used to reduce the pH of the fermentation broth. No additional acid is needed for controlling the pH of the fermentation broth. In addition, when ammonia, ammonium hydroxide and/or an ammonium salt is used as the nitrogen source, pH of the fermentation broth may be increased. No additional base is needed for controlling the pH. At least in some embodiments of the present disclosure, a composition comprising the carbon source and the nitrogen source in desired ratio is formed. The composition may then be added to the fermentation broth or to the fermentation medium. For example, when the carbon and the nitrogen source are added to an aqueous solution in a molar ratio of 20:1 respectively, the pH of the solution may be around 5. In other examples, the carbon source and the nitrogen source may be added to an aqueous solution in a molar ratio of 90:1 respectively, yielding in a solution with pH in the range of 3-4. These solutions can then be used in some examples for controlling the pH of the fermentation broth.

Typically, the pH of the fermentation broth is monitored. Monitoring may be performed by any direct or indirect measurement of pH, for example by using a pH sensor. The system may be operated for example so that when the pH raises above a predetermined threshold, an amount of the carbon source and nitrogen source is added so that the pH of the fermentation broth is corrected back to the desired level. Alternative monitoring strategies may be also be used, for example direct or indirect measurement of the acid and/or nitrogen content of the fermentation broth.

Different compositions comprising the carbon source and the nitrogen source may be used at different stages of the fermentation. In a typical fermentation process a microbial biomass is produced first. After a sufficient amount of microbial biomass has been produced, production of polyhydroxyalkanoates is induced. By using different ratio of the carbon source and the nitrogen source in primary bioreactor(s) than in the secondary bioreactors, such induction of production of polyhydroxyalkanoates can be obtained. In other words, it has been observed that providing a higher carbon to nitrogen ratio in the secondary bioreactors than in the at least one primary bioreactor induces production of polyhydroxyalkanoates. Thus, in a typical fermentation process according to the present disclosure, the carbon source and nitrogen source are added at lower carbon to nitrogen ration to the primary bioreactor than to the secondary bioreactors. In some examples, the carbon source and the nitrogen source may be introduced using the same ratio for all primary bioreactors and using another ratio for all secondary bioreactors. In other examples, the carbon source and the nitrogen source may be introduced at different ratio to each secondary bioreactor. Surprisingly, it has been observed that some nitrogen has to be introduced also to the secondary bioreactors for optimal production of polyhydroxyalkanoates. In some embodiments, the carbon source and the nitrogen source may be added to the at least one primary bioreactor in a molar ratio ranging from 10:1 to 25:1, for example in a molar ratio ranging from 15:1 to 20:1. In some embodiments the carbon source and the nitrogen source may be added to the secondary bioreactors in a molar ratio ranging from 70:1 to 120:1, for example a molar ratio ranging from 80:1 to 100:1. For example, the carbon source and the nitrogen source may be added to the at least one primary bioreactor in a molar ratio ranging from 10:1 to 25:1 and to the secondary bioreactors in a molar ratio ranging from 70:1 to 120:1 or in a molar ratio ranging from 80:1 to 100:1. Such ratios have been observed to also provide sufficient amounts of carbon and nitrogen for biomass and polyhydroxyalkanoate production.

The herein disclosed method allows simultaneous adjustment of the pH of the fermentation broth and control of the stage of the fermentation process because the acids used for correcting the pH are simultaneously used as the carbon source. Thus, it has been observed that pH control may be repurposed to control feeding of the carbon source during the fermentation process. The pH of a composition with a lower carbon to nitrogen ratio, e.g. C:N molar ratio of 5:1 to 30:1, is higher than the pH of a composition with a higher carbon to nitrogen ratio, e.g. C:N molar ratio 70:1 to 150:1. Thus less of a composition comprising a higher C:N ratio is needed to adjust the pH of the fermentation broth to the same extent as when using a composition comprising a lower C:N ratio.

The pH of the fermentation broth is maintained in the range of 6 to 8. The pH range is typically adjusted so that it is optimal for the growth of the micro-organisms comprised in the biomass and/or for the production of at least one polyhydroxyalkanoate. Many micro-organisms prefer a neutral pH. Thus, the pH maybe maintained in some embodiments in the range of 6.5 to 7.5. It is possible to use a different pH for the at least one primary bioreactor and for the secondary bioreactors. In many embodiments however, the pH is kept at a certain range throughout the fermentation process.

In some embodiments, the volatile fatty acid may be selected from acetic acid, propionic acid, butyric acid, valeric acid, isovaleric acid, succinic acid, hexanoic acid, or combinations thereof. It is possible to use also salts or esters of volatile fatty acids as the carbon source. Examples of salts of volatile fatty acids include acetate, propionate, butyrate, valerate, isovalerate, and hexanoate. The mixture of volatile fatty acids may comprise two or more of acetic acid, propionic acid, butyric acid, valeric acid, isovaleric acid, or hexanoic acid. A large amount of acids in the fermentation broth may be inhibitory to the micro-organisms comprised in the biomass. Thus, at least in some embodiments the total amount of volatile fatty acids is less than 10 g/L in the fermentation broth at any point during the fermentation process. This may be achieved by dosing the volatile fatty acids in a way that keeps their concentration in the reactors at a level that is not inhibitory to the micro-organism at all times. For example, pH of the fermentation broth may be corrected by multiple additions of small amounts of the carbon source and nitrogen source instead of adding a large amount of the carbon source and the nitrogen source at one time point.

In principle, other organic acids or fatty acids could also be used as the carbon source. Such other fatty acids include for example fatty acids with 7 to 10 carbon atoms, such as heptanoic acid or octanoic acid, but also for example vegetable oils. However, shorter fatty-acids, such as volatile fatty acids, can be produced from CO₂ and thus using them as a carbon source allows production of bioplastic from carbon dioxide waste making the process more environmentally sustainable.

In some embodiments, the mixture of volatile fatty acids may comprise at least 1 %, such as at least 5 %, of valeric acid, isovaleric acid, and/or hexanoic of the weight of the mixture of volatile fatty acids.

In some embodiments, the mixture of volatile fatty acids may be produced from carbon dioxide and hydrogen gas by using at least one microorganism expressing the enzymes of the Wood-Ljungdahl pathway. The carbon dioxide may be obtained from incineration or combustion of waste material or from industrial processes, such as pulp and paper industry. In this way, bioplastics can be produced from carbon dioxide waste. The Wood-Ljungdahl pathway, also known as reductive acetyl-coenzyme A (acetyl-CoA) pathway, allows microorganisms to use hydrogen (H₂) as an electron donor and carbon dioxide (CO₂) as an electron acceptor. Carbon dioxide is used as a carbon source by these microbes. The Wood-Ljungdhal pathway is characterized by the use of hydrogen as an electron donor and carbon dioxide as an electron acceptor to produce acetyl-CoA as the final product. The enzymes of Wood-Ljungdhal pathway attaches two carbon units derived from carbon dioxide directly to each other rather than adding carbon dioxide to an already formed carbon chain. Two specific enzymes are required for the pathway: carbon monoxide dehydrogenase and acetyl-CoA synthase. The former catalyses the reduction of CO₂ and the latter combines the resulting CO with a methyl group, which has also been formed from CO₂, to give acetyl-CoA. In addition to these two specific enzymes, many other enzymes are required for the pathway to be functional. Microorganisms expressing the enzymes of the Wood-Ljungdahl pathway comprise for example acetogens, which typically are obligately anaerobic bacteria. The term "acetogens" refers herein to microorganisms that produce acetate as an end product of anaerobic respiration or fermentation. In some examples, the microorganisms expressing the enzymes of the Wood-Ljungdahl pathway comprise or consists of homoacetogens. Homoacetogens may produce acetyl-CoA, and from that, in most cases, acetate as the end product, from two molecules of carbon dioxide (CO₂) and four molecules of molecular hydrogen (H₂). Examples of microorganisms expressing the enzymes of the Wood-Ljungdahl pathway comprise bacteria belonging to the genus *Pseudomonas, Clostridium, Moorella, Citrobacter, Acetobacter, Cupriavidus, Calderihabitans, Carboxydothermus,* or *Defluviitoga.*

In some embodiments, the production of volatile fatty acids by using at least one microorganism expressing the enzymes of the Wood-Ljungdahl pathway may be performed at a temperature of below 20 °C, preferably at a temperature in the range of 8 °C to 18 °C. Using low temperatures for production of volatile fatty acids affects the type of volatile fatty acids that are produced. At lower temperatures, the amount of produced acetic acid typically decreases while the amount of other volatile fatty acids increases. For example, the amount of valeric acid, isovaleric acid and/or hexanoic acid may increase when the production of volatile fatty acids is performed at low temperatures.

The nitrogen source in the herein disclosed fermentation process is ammonia, ammonium hydroxide and/or an ammonium salt. Ammonia may be provided together with an aqueous solution, such as water. The ammonium salt may in some examples be an ammonium salt of a volatile fatty acid. Such ammonium salts may include for example ammonium acetate, ammonium propionate, ammonium butyrate, ammonium valerate, and/or ammonium hexanoate. In some embodiments, a mixture of different ammonium sources may be used, for example a mixture of ammonium hydroxide and ammonium acetate. Some ammonium salts may be less desirable than others because of technical issues. Such less desirable ammonium sources include for example ammonium chloride, ammonium nitrate and ammonium sulphate.

In some embodiments the volatile fatty acid and the ammonia, ammonium hydroxide and/or ammonium salt are the sole carbon source and the sole nitrogen source for the biomass, respectively. Such set up is advantageous because if other carbon and/or nitrogen sources are available, the control of pH by using the carbon source and/or the nitrogen source might be difficult or even impossible to accomplish. However, small amounts of other carbon and/or nitrogen sources may be in some embodiments present without harmfully affecting the overall process. For example, the microbial biomass may produce small amounts of other carbon sources and/or nitrogen sources or the supplemented fermentation medium may contain residual amounts or be supplemented with an initial concentration of other carbon and/or nitrogen sources. Preferably however, other carbon and nitrogen sources are excluded from the process or the amount of other carbon and nitrogen sources is less than 0.01 wt-% during the fermentation process.

A variety of suitable fermentation media are well known in the art and can be selected by one having ordinary skill in the art, depending upon which microorganism(s) is used. Generally, it is required that a suitable fermentation medium is able to provide the chemical components necessary to maintain metabolic activity and/or to allow cell growth. In the present disclosure such fermentation media is however depleted of any carbon source and of any nitrogen source. In some embodiments, a fermentation medium comprising metal ions, and salts and depleted of any carbon source or nitrogen source may be provided as a feed separate from the carbon source and the nitrogen source. In some examples, the fermentation medium does not contain any organic compounds, such as for example organic acids. For example, the fermentation medium may comprise various metal ions at concentration below 10 mg/L each and salts of Mg, Na, K, SO₄ and/or PO₄. One advantage with having the carbon source and the nitrogen source as a separate feed to other components needed for the fermentation process is that it allows providing required amount of nutrients independently of the amount of needed carbon source or nitrogen source. This simplifies process optimization. Another advantage is that such approach enables controlling contamination, which especially in continuous fermentation processes presents a challenge. Microbes do not grow in a fermentation medium that is depleted of a carbon source and a nitrogen source because both elements are needed for microbial growth. On the other hand, a composition comprising high amounts of volatile fatty acids as carbon source are very acidic thus preventing micro-organisms from growing. By providing the carbon source and nitrogen source as a separate feed from other nutrients, a composition with high acid content can be produced. For example, the carbon source and the nitrogen source may be provided as a solution comprising at least 30 %, such as at least 50 %, of at least one volatile fatty acid from the weight of the solution. In some examples, the carbon source and the nitrogen source may be added at the same time or at separate time points with the fermentation medium to the primary bioreactor(s). In other examples, the carbon source and the nitrogen source may be added first to the fermentation medium and the fermentation medium may shortly thereafter be added to the primary bioreactor(s). Fermentation medium is typically not added to the secondary bioreactors. Typically, enough nutrients are provided when the fermentation broth is passed from the at least one primary bioreactor to the secondary bioreactors. However, in some embodiments, the fermentation medium may also be added to the secondary bioreactors. The composition of the fermentation medium is typically such that addition of the fermentation medium does not affect the pH of the fermentation broth, at least not to any large extent. The pH of the fermentation medium may be for example about 7, for example in the range of 6.5 to 7.5.

The process disclosed herein is a continuous fermentation process. Thus, in some embodiments the process is configured to maintain a continuous flow from a preceding bioreactor to a subsequent bioreactor. In some embodiments, such configuration is obtained by continuously extracting the fermentation broth during or after the formation of at least one polyhydroxyalkanoate at a rate allowing maintaining the volume of the fermentation broth in the at least one primary bioreactor and in the secondary bioreactors at constant or close to constant. The bioreactors may for example comprise pumps with actuators that enable setting minimum and/or maximum value to take out a desired volume per unit time. At the same time fermentation medium and/or the carbon source and nitrogen source are introduced to the bioreactors using the same desired volume per unit time. Typically, the system is not at a perfect steady state but may have a continuous flow with some variation in volume over a short period of time. In other words, about 5 % fluctuation of the volume in a bioreactor may in some examples be observed during the process. In some embodiments, the fermentation broth may be continuously extracted from at least one of the secondary bioreactors during or after the formation of the at least one polyhydroxyalkanoate at a rate allowing the volume of the fermentation broth in the at least one primary bioreactor and in the secondary bioreactors to change less than 5 % of the initial volume of the bioreactors. Initial volume refers here to the volume of fermentation medium provided to the bioreactors before or at the start of the fermentation process.

Biomass in mainly produced in the at least one primary bioreactor. However, some biomass production may take place also in the secondary bioreactors. In some embodiments, the biomass may be produced in one primary bioreactor. In other examples, multiple primary bioreactors, for example 2 to 4 primary bioreactors, may be used to produce the biomass. The biomass produced in multiple primary bioreactors may be combined prior to passing it through the secondary bioreactors or it can be directly passed to secondary bioreactors from the multiple primary bioreactors. In at least some preferred embodiments, the at least one primary bioreactor is inoculated with a starter culture comprising the at least one micro-organism capable of producing the at least one polyhydroxyalkanoate at the beginning of the fermentation process. Preferably, new inoculation is not performed in secondary bioreactors but the biomass in the secondary bioreactors originates from the at least one primary bioreactor.

After formation of the biomass, the fermentation broth is passed though secondary bioreactors. The system may have as many secondary bioreactors as desired. Typically, a change in the formed product can be observed in each secondary bioreactor because the conditions within each secondary bioreactor differs from each other. In the first secondary bioreactor, the fermentation broth comprises a relatively high amount of the nitrogen source and other nutrients because these were provided to the primary bioreactor(s) from which the fermentation broth is then transferred to the first secondary bioreactor. In each of the following bioreactors the amount of nitrogen and nutrients decreases because the carbon to nitrogen ratio of the feed increases. Further, typically the fermentation medium is not introduced to the secondary bioreactors. Thus, the composition of the fermentation broth changes the more secondary bioreactors are added. In some embodiments, the fermentation broth is passed through two to five secondary bioreactors. It has been observed, that 2-5 secondary bioreactors may allow production of polyhydroxyalkanoates with good quality. The yield of produced polyhydroxyalkanoate is not high if only one secondary bioreactor is used because the amount of the nitrogen source is too high to sufficiently induce polyhydroxyalkanoate production. In principle, the more secondary bioreactors the system has the better control of the polyhydroxyalkanoate production can be obtained. However, more than five secondary bioreactors will increase the production cost of the polyhydroxyalkanoate to a level that is not optimal for subsequent uses of the bioplastic. In some embodiments, the secondary bioreactors may be organized in series, such as in linear series. The fermentation broth may be in some embodiments passed from the at least one primary bioreactor only to a first secondary bioreactor in the series. From the first secondary bioreactor the fermentation broth may then be passed through the following secondary bioreactors of the series. In other embodiments, at least some of the fermentation broth may be passed from the at least one primary bioreactor to two or more of the secondary bioreactors.

The fermentation broth may be continuously extracted from the secondary bioreactors during or after formation of the at least one polyhydroxyalkanoate. In some embodiments, the fermentation broth is extracted only from the last secondary bioreactor of the series. In some embodiments, the biomass may be separated from the fermentation broth after extracting the fermentation broth from the secondary bioreactors. Separation may be performed by any suitable method, for example by filtration or centrifugation.

The process may be performed in a system comprising several separate bioreactors that are connected to each other for example by using conduits between the bioreactors. Each bioreactor may be an aerobic fermenter including one or more vessels, towers, or other suitable containers configured to accommodate fermentation medium. Typically, the primary bioreactor may comprise a conduit configured to permit efflux to continuously flow to a secondary bioreactor. Secondary bioreactor may be configured to continuously receive efflux from the primary bioreactor or from another secondary bioreactor. In addition, the secondary bioreactors may be configured to permit efflux to continuously flow to other secondary bioreactors. For example, the bioreactors may be arranged in series such that the effluent of the first bioreactor (primary bioreactor) is fed to a second bioreactor in series (first secondary bioreactor) and the effluent of the second bioreactor is fed to a third bioreactor in the series (second secondary bioreactor), and so forth until the last bioreactor of the series is reached.

Each bioreactor may comprise means for monitoring the fermentation process. Such means may include for example a pH sensor, a dO₂ sensor, a CO₂ sensor, a pressure sensor, or any combinations thereof. The bioreactors may be configured to monitor and adjust the pH of the fermentation broth by using the carbon source and the nitrogen source. For example, a bioreactor may be connected to a pH control system. A pH control system can include sensors for detecting pH and/or optical density of the fermentation broth and a liquid handling system enabling the addition of the carbon source, the nitrogen source and/or of the fermentation medium in response to variances in the pH relative to a pre-set value. This can be achieved for example by using dosing pumps.

Bioreactors may be configured to permit monitoring, controlling and/or adjusting one or more of the following conditions within the bioreactors: temperature, pH, foaming, optical density, pressure, CO₂, and dissolved oxygen (dO₂). Systems permitting monitoring, controlling, and/or adjusting may include one or more computer processors, a user interface for displaying data or for receiving user input, and memory containing computer readable instructions to be executed by the processors and/or for storing data.

Secondary bioreactors may be configured to permit continuous extraction of the fermentation broth from the bioreactor during or after formation of the at least one polyhydroxyalkanoate. Configurations for extraction may include ports or other access points enabling removal of the fermentation broth.

The fermentation process disclosed herein produces at least one polyhydroxyalkanoate. In some embodiments, the at least one polyhydroxyalkanoate is formed by the polymerization of 3-hydroxyvalerate, 3-hydroxybutyrate, 3-hydroxyhexanoate, 3-hydroxypropionate, or combinations thereof. The at least one polyhydroxyalkanoate may comprise or consist of poly(3-hydroxybutyrate-co-3-hydroxyvalerate) (PHBV), poly-3-hydroxyvalerate or combinations thereof.

Another aspect of the present disclosure is a product obtainable with the process disclosed herein. Such product comprises the at least one polyhydroxyalkanoate. The amount of the at least one polyhydroxyalkanoate may in some embodiments be at least 50 % of the dry weight of the product. In other words, the biomass in secondary bioreactors accumulates at least 50 % of polyhydroxyalkanoate of the cell dry weight.

### Examples

### Example 1

### Material and chemicals

The bacterium Cupriavidus necator DSM 545 was used for the study.

Composition of the main feed medium - KH₂PO₄ 6 g/L, MgSO₄•7H₂O 0.5 g/L, and trace metal salts - FeSO₄•7H₂O 50 mg/L, ZnSO₄•7H₂O 22.5 mg/L, CuCl₂ 2H₂O 6.8 mg/L, MnSO₄ H₂O 3.5 mg/L, CaSO₄ 2H₂O 23.4 mg/L, Na₂B₄O₇•10H₂O 5 mg/L, 35% HCl - 0.1 mL/L. The pH of the medium was adjusted to 6.8 using NaOH.

The CN20 solution comprising carbon and nitrogen in molar ratio of 20:1 was prepared by adding 666 mL of a mixture of volatile fatty acids (VFA) together with 146.1 mL of 35% ammonium hydroxide and water. The mixture of volatile fatty acids included acetic acid, propionic acid, butyric acid and valeric acid. The pH of CN 20 solution was 5.09. Similarly, the CN90 solution comprising carbon and nitrogen in molar ratio of 90:1 was prepared by mixing 666 mL of the above mentioned VFA mixture together with 32.5 mL of 35% ammonium hydroxide and water. The pH of CN90 solution was 3.60.

The VFA were analyzed using an organic acid analysis kit LCK 365 and were measured by HACH DR6000 spectrophotometer. Similarly, the ammonium concentration was measured using the kit LCK 305 or LCK 502. The cell growth was measured by measuring the absorbance of the culture at 600 nm wavelength. The PHA concentration was measured by using thermogravimetric analysis. Moreover, Fourier-transform infrared spectroscopy analysis of the freeze-dried cells was performed to assess the presence of PHA in the cells.

### Arrangement and operation of reactors

The bioreactors were operated at 30 °C in a continuous mode with dilution rate between 0.05 to 0.055. The bioreactors were continuously stirred at 1000 rpm and were sparged with O₂ at 0.2 to 0.5 vvm. The reactors were arranged in series such that the effluent of 1st reactor (R1) is fed to the reactor 2 (R2) in series and the effluent of R2 is fed to the 3rd reactor in the series (R3). The pH in the reactor R1 was controlled at 6.8 to 7.0, R2 6.9 to 7.1 and R3 6.9 to 7.1 by controlled addition of CN solutions. For R1, CN20 was used and for R2 and R3 CN90 was used. The difference in the pH in the reactors and the CN solutions was used to control the pH in reactors as well as to add the carbon and nitrogen in controlled manner to minimize substrate inhibition. The peristaltic pumps that were used to add the CN solutions were programmed to limit the dosage of CN solutions between a predetermined range to control the dosing.

### Results

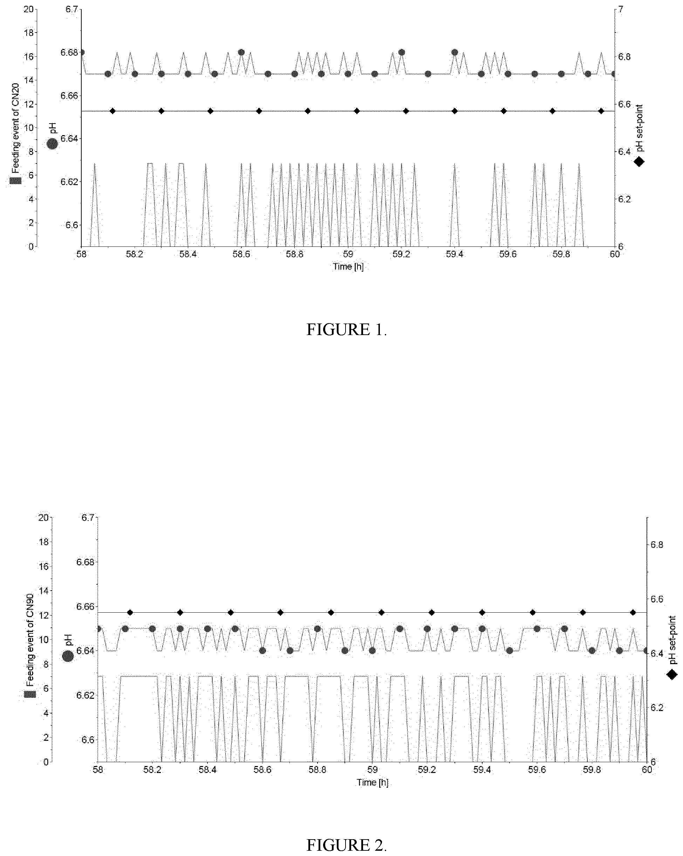

Of the three reactors arranged in series, R1 functioned as a primary bioreactor producing biomass with OD600 reaching to 38. The carbon and nitrogen source required for the growth in R1 was provided by dosing CN20 solution that was triggered by change in pH in the reactor. An example of CN20 based pH control in R1 is shown in Figure 1. Furthermore, the reactors R2 and R3 facilitated accumulation of PHA by using the carbon source provided via dosing of CN90 solution which was triggered by change in pH in respective reactors. An example of CN90 based pH control in R2 is shown in Figure 2. In both cases, the CN solution pump is activated when the pH increases above the set-point (deadband 0,1) to maintain the pH set-point. The dosing of CN 20 in R1 varied from 3 to 9 mL/L/h. Similarly, the dosing of CN90 in R2 and R3 varied between 1 to 10 ml/L/h and 1 to 7 mL/L/h, respectively. The concentration of VFA in R1, R2 and R3 did not exceed to more than 4.7, 5.1, 4.2 g/L, respectively. Additions of the CN solutions described herein were sufficient for controlling the pH of the fermentation broth at desired level throughout the fermentation process. No additional agents for adjusting the pH were needed.

Approximately 594 g of wet cell mass was collected during 3,5 days from the reactor R3. The cells were dried using a freeze-dryer resulting in a dry biomass weight of 245 g. Quantification analysis using thermogravimetric analysis (TGA) determined PHA content to be 59 % within the cells. In further processing, the PHA was extracted from the lyophilized biomass samples resulting in a 77,5 w/w % PHA yield with 94 % purity.

### Experiment 2

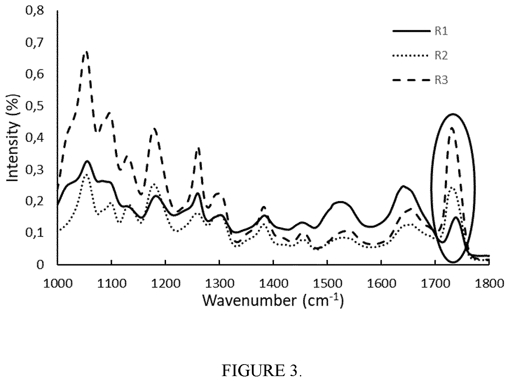

In a second experiment, Fourier-transform infrared spectroscopy (FTIR) was used to quantify PHA content of the biomass in the primary (R1) and secondary (R2 and R3) reactors (Figure 3). The characteristics of PHA FTIR absorbance profile can include peaks at wavenumbers 1728 cm⁻¹, 1736 cm⁻¹, 1744 cm⁻¹, 1280 cm⁻¹, 1000-1200 cm⁻¹, where 1728 cm⁻¹ and 1744 cm⁻¹ peaks represent the ester carbonyl group C=O most essential for PHA (Ziganova et al). The FTIR spectra demonstrate the significant absorbance at the wavenumbers specified above and thus strongly indicate the accumulation of PHA in reactor R2 and R3. The distinctive peaks at 1637 cm⁻¹ and at 1536 cm⁻¹ represent amide bonds in proteins (Kansiz et al). Thus, the absorbance at these wavelengths represent the 'non-PHA biomass'. The spectra in Fig. 3 clearly indicates the higher biomass in R1 compared to R2 or R3. Moreover, it also clearly indicates the higher PHA concentration in R2 and R3 compared to R1. Further, the amount of PHA increased from R2 to R3 due to the fact that less carry over from R1 was present in R3 than in R2.

It is to be understood that the embodiments of the invention disclosed are not limited to the particular structures, process steps, or materials disclosed herein, but are extended to equivalents thereof as would be recognized by those ordinarily skilled in the relevant arts. It should also be understood that terminology employed herein is used for the purpose of describing particular embodiments only and is not intended to be limiting.

Reference throughout this specification to "one embodiment" or "an embodiment" means that a particular feature, structure, or characteristic described in connection with the embodiment is included in at least one embodiment of the present invention. Thus, appearances of the phrases "in one embodiment" or "in an embodiment" in various places throughout this specification are not necessarily all referring to the same embodiment.

As used herein, a plurality of items, structural elements, compositional elements, and/or materials may be presented in a common list for convenience. However, these lists should be construed as though each member of the list is individually identified as a separate and unique member. Thus, no individual member of such list should be construed as a de facto equivalent of any other member of the same list solely based on their presentation in a common group without indications to the contrary. In addition, various embodiments and example of the present invention may be referred to herein along with alternatives for the various components thereof. It is understood that such embodiments, examples, and alternatives are not to be construed as de facto equivalents of one another, but are to be considered as separate and autonomous representations of the present invention.

Furthermore, the described features, structures, or characteristics may be combined in any suitable manner in one or more embodiments. In the following description, numerous specific details are provided, such as examples of lengths, widths, shapes, etc., to provide a thorough understanding of embodiments of the invention. One skilled in the relevant art will recognize, however, that the invention can be practiced without one or more of the specific details, or with other methods, components, materials, etc. In other instances, well-known structures, materials, or operations are not shown or described in detail to avoid obscuring aspects of the invention.

While the forgoing examples are illustrative of the principles of the present invention in one or more particular applications, it will be apparent to those of ordinary skill in the art that numerous modifications in form, usage and details of implementation can be made without the exercise of inventive faculty, and without departing from the principles and concepts of the invention. Accordingly, it is not intended that the invention be limited, except as by the claims set forth below.

The verbs "to comprise" and "to include" are used in this document as open limitations that neither exclude nor require the existence of also un-recited features. The features recited in depending claims are mutually freely combinable unless otherwise explicitly stated. Furthermore, it is to be understood that the use of "a" or "an", i.e. a singular form, throughout this document does not exclude a plurality.

### REFERENCES

Kansiz M, Billman-Jacobe H, McNaughton D 2000. Quantitative Determination of the Biodegradable Polymer Poly(β-hydroxybutyrate) in a Recombinant Escherichia coli Strain by Use of Mid-Infrared Spectroscopy and Multivariative Statistics. Appl Environ Microbiol 66:. https://doi.org/10.1128/AEM.66.8.3415-3420.2000
Žiganova, M.; Merijs-Meri, R.; Zicaa̅ns, J.; Bochkov, I.; Ivanova, T.; Vi̅gants, A.; Ence, E.; Štrausa, E. Visco-Elastic and Thermal Properties of Microbiologically Synthesized Polyhydroxyalkanoate Plasticized with Triethyl Citrate. Polymers 2023, 15, 2896. https://doi.org/10.3390/polym15132896

## Claims

1. A continuous fermentation process for producing at least one polyhydroxyalkanoate, comprising the steps of:
- producing a fermentation broth comprising a biomass in at least one primary bioreactor;
- passing the fermentation broth through secondary bioreactors for producing at least one polyhydroxyalkanoate;
wherein
- the biomass comprises at least one micro-organism capable of producing the at least one polyhydroxyalkanoate;
- pH within the bioreactors is maintained in the range of 6 to 8 by addition of a carbon source and a nitrogen source, wherein
• the carbon source is a volatile fatty acid or a mixture of volatile fatty acids, and the nitrogen source is ammonia, ammonium hydroxide, an ammonium salt or a combination thereof;
• the carbon source and the nitrogen source are added to the at least one primary bioreactor in a molar ratio ranging from 5:1 to 30:1, and to the secondary bioreactors in a molar ratio ranging from 70:1 to 150:1.

2. The process according to claim 1, wherein the volatile fatty acid is selected from acetic acid, propionic acid, butyric acid, valeric acid, isovaleric acid, hexanoic acid, or combinations thereof.

3. The process according to any of the preceding claims, wherein the volatile fatty acid and the ammonia, ammonium hydroxide, ammonium salt or combination thereof are the sole carbon source and the sole nitrogen source, respectively, for the at least one micro-organism.

4. The process according to any of the preceding claims, wherein a fermentation medium comprising metal ions and salts and depleted of the carbon source and the nitrogen source is introduced as a feed separate from the carbon source and the nitrogen source.

5. The process according to any of the preceding claims, wherein the total amount of volatile fatty acids is less than 10 g/L in the fermentation broth at any point during the fermentation process.

6. The process according to any of the preceding claims, wherein the carbon source and the nitrogen source are added as a solution comprising at least 30 %, such as at least 50 % of at least one volatile fatty acid of the weight of the solution.

7. The process according to any of the preceding claims, wherein the fermentation broth is passed through two to five secondary bioreactors.

8. The process according to any of the preceding claims, wherein the process is configured to maintain a continuous flow from a preceding bioreactor to a subsequent bioreactor.

9. The process according to any of the preceding claims, wherein the fermentation broth is continuously extracted from at least one of the secondary bioreactors during or after the formation of the at least one polyhydroxyalkanoate at a rate allowing the volume of the fermentation broth in the at least one primary bioreactor and in the secondary bioreactors to change less than 5 % of the initial volume in the bioreactors.

10. The process according to claim 9, wherein the biomass is separated from the fermentation broth after the extraction from the at least one secondary bioreactor.

11. The process according to any of the preceding claims, wherein the at least one polyhydroxyalkanoate is formed by the polymerization of 3-hydroxyvalerate, 3-hydroxybutyrate, 3- hydroxyhexanoate, 3-hydroxypropionate, or combinations thereof.

12. The process according to any of the preceding claims, wherein the mixture of volatile fatty acids comprises at least 1 %, such as at least 5 %, of valeric acid, isovaleric acid, and/or hexanoic acid of the weight of the mixture of volatile fatty acids.

13. The process according to any of the preceding claims, wherein the mixture of volatile fatty acids is produced from carbon dioxide and hydrogen gas by using at least one microorganism expressing the enzymes of the Wood-Ljungdahl pathway.

14. The process according to any of the preceding claims, the carbon source and the nitrogen source are added to the at least one primary bioreactor in a molar ratio ranging from 10:1 to 25:1 and to the secondary bioreactors in a molar ratio ranging from 70:1 to 120:1.

15. A product obtainable with the process according to any of the preceding claims.
